# EUROPEAN PATENT APPLICATION

(11) **EP 2 201 953 A1**
(43) Date of publication of application: **30.06.2010**
(21) Application number: 08828257.9
(22) Date of filing: 27.08.2008
(51) Int. Cl.: A61K 31/7056, A61K 38/21, A61P 7/04, A61P 31/12

(54) **USE OF RIBAVIRIN IN BLOOD COAGULATION DISORDER**

(30) Priority: 27.08.2007 JP 2007220449; 27.08.2007 JP 2007220450
(71) Applicant: National University Corporation Nagoya University, Nagoya-shi, Aichi 464-8601 (JP)
(72) Inventor: HONDA, Takashi, Nagoya-shi Aichi 464-8601 (JP); TAKAMATSU, Junki, Nagoya-shi Aichi 464-8601 (JP); TOYODA, Hidenori, Nagoya-shi Aichi 464-8601 (JP); YAMAMOTO, Koji, Nagoya-shi Aichi 464-8601 (JP); GOTO, Hidemi, Nagoya-shi Aichi 464-8601 (JP)
(74) Representative: Woods, Geoffrey Corlett
(86) International application number: PCT/JP2008/065338
(87) International publication number: WO 2009/028573

(57) **Abstract**

To provide a pharmaceutical agent that is effective for the amelioration or the like of a condition where the prothrombin time is prolonged or a condition where the INR (international normalized ratio) is increased. Disclosed is a pharmaceutical agent comprising ribavirin or a derivative thereof as an active ingredient.

## Description

### Technical Field

The present teaching relates to the use of ribavirin in blood coagulation disorders. More specifically, the teaching relates to a pharmaceutical agent having a prothrombin time shortening activity or an INR elevating activity, and the use thereof.

### Background Art

Platelets and various blood coagulation factors take part in the coagulation of blood. Known blood coagulation factors include blood coagulation factors I to XIII, the von Willebrand factor, and the Fitzgerald factor. Many of these coagulation factors, including factors I, II, V, VIII, and IX to XIII, are produced in the liver. When such blood coagulation factors are congenitally deficient or the levels thereof in the blood are low due to some acquired effect, this leads to the onset of a blood coagulation disorder. "Blood coagulation disorder" refers herein to a blood disease which causes anomalies in the hemostatic system and/or coagulation system and manifests a bleeding tendency. Typical examples of blood coagulation disorders include hemophilia A which is characterized by a deficiency of blood coagulation factor VIII, and hemophilia B which is characterized by a deficiency of blood coagulation factor IX. The treatment of blood coagulation disorders generally involves carrying out replenishment therapy to replenish the deficient coagulation factors.

It has been reported that ribavirin and interferon, both known as antiviral agents which are nucleoside derivatives, increase the amount of coagulation factor VII in blood when administered to hemophilia patients (Non-Patent Document 1). Although factor VII is not a coagulation factor that is deficient in hemophilia A and hemophilia B, by administering factor VII, fibrin can be fibrosed without involving the activation of factors VIII and IX in the blood coagulation cascade. Factor VII administration is thus called a "bypass" therapy. Moreover, when ribavirin and interferon are administered to hemophilia patients who have contracted the hepatitis C virus, the level of von Willebrand factor in the blood is known to increase (Non-Patent Document 2).

When administered such replenishment therapy, there are some cases in which the patient develops antibodies (inhibitors) to the replenished coagulation factors. In such inhibitor carriers, even when the deficient coagulator factors are replenished, operation of the coagulation factors is ultimately blocked (neutralized) by the appearance of inhibitors, thereby making hemostasis difficult to control.

The method used to treat blood coagulation disorders in inhibitor carriers generally involves administering activated blood coagulation factor VII in order to increase the hemostatic effects due to extrinsic blood coagulation. The administration of blood coagulation factor X as the main active ingredient, and the administration of a combination of activated blood coagulation factor VII and blood coagulation factor X have also been disclosed (Patent Documents 1 and 2).

Patent Document 1: Japanese Patent Application Publication No. 2007-55899
Patent Document 2: Japanese Patent Application Publication No. 2001-181204
Non-Patent Document 1: Journal of Thrombosis and Haemostasis 4, 469-487
Non-Patent Document 2: Aliment. Pharmacol. Ther. 21, No. 1, 49-55 (Jan. 1, 2005)

### Disclosure of Invention

However, it is not known, nor could it have been anticipated even by persons of ordinary skill in the art, that the levels of coagulation factors other than coagulation factor VII and the von Willebrand factor increase with the use of ribavirin alone or the concomitant use of ribavirin and interferon.

Were it possible to increase the levels of blood coagulation factors other than coagulator factor VII and the von Willebrand factor, a novel treatment could be provided as an alternative to replenishment therapy for congenital or acquired deficiencies of these other blood coagulation factors. A therapy effective for ameliorating conditions in which the prothrombin time is prolonged or conditions in which the international normalized ratio (INR) is elevated could also be provided. Moreover, a novel drug to prevent or stop bleeding during a surgical procedure or when bleeding occurs due to an external injury or a disease other than a blood coagulation disorder, such as a cerebral hemorrhage, could additionally be provided.

Although the risk of virus contamination in coagulant factor preparations has been greatly reduced, when blood preparations are used, the possibility of virus infection cannot be entirely dismissed. Also, even though there is a high probability that the risk of virus infection can be avoided by using a recombinant preparation produced by genetic engineering, such preparations are very expensive and thus impose a large financial burden on the patient.

In addition, taking into account the fact that inhibitor carriers develop inhibitors to the deficient coagulation factors that have been replenished, even in cases where types of coagulation factors differing from the deficient coagulation factors have been administered to an inhibitor carrier, the possibility that effects which lower or suppress in some way the operation of the coagulation factors will emerge cannot be entirely dismissed. Moreover, because the blood coagulation factors administered are not autogenous, the possibility that they may have some kind of adverse effect on the body also cannot be dismissed.

Hence, in spite of all its drawbacks, the only therapy currently available for ameliorating bleeding tendencies and preventing bleeding in blood coagulation disorders is replenishment therapy. Moreover, because replenishment therapy in an inhibitor carrier includes the possibility that additional antibodies will develop, this has been a treatment mode to be avoided where at all possible. Yet, in spite of this, new pharmaceutical agents which are effective in inhibitor carriers and can be used in place of coagulation factor replenishment have not been provided to date.

Therefore, an object of the teaching is to provide a drug which is effective for diseases or symptoms related to blood coagulation factors and can be used instead of the external replenishment of blood coagulation factors. Another object of the teaching is to provide a drug effective for ameliorating conditions in which the prothrombin time is prolonged or conditions in which the international normalized ratio (INR) is elevated. Yet another object of the teaching is to provide a non-replenishment pharmaceutical agent which is effective for the amelioration or replenishment of blood coagulation factor II, V, and VIII deficiencies.

Another object of the teaching is to provide a pharmaceutical agent which is capable of ameliorating bleeding tendencies and preventing bleeding in blood coagulation disorder patients who carry inhibitors, without the external replenishment of blood coagulation factors.

The inventors have learned that when ribavirin is administered alone in hemophilia patients, there is a tendency for the patient's serum during the period of administration to undergo a shortening in the prothrombin time (for the PT% to rise) and a decline in the INR. They have also learned that when ribavirin and interferon are administered for the purpose of treating chronic hepatitis C in patients who have hemophilia and chronic hepatitis C, there is a tendency for the patient's serum during such administration to undergo a shortening in the prothrombin time and a decline in the INR. At the same time, they have found that such administration increases the levels of a plurality of blood coagulation factors other than blood coagulation factor VII and the von Willebrand factor, for which such increases have hitherto been known. In addition, the inventors have discovered that when ribavirin is administered alone in hemophilia patients, the amount of coagulant used decreases dramatically. Based on these findings, the inventors ultimately arrived at the present teaching, which is recited below.

The teaching provides a pharmaceutical agent which includes ribavirin or a derivative thereof as an active ingredient, the pharmaceutical agent ameliorating, preventing or treating a condition in which a prothrombin time is prolonged or a condition in which an international normalized ratio (INR) is elevated. This pharmaceutical agent may be obtained in a form in which ribavirin or a derivative thereof is combined with interferon. The pharmaceutical agent may be used in particular to ameliorate, prevent or treat a condition in association with liver disease in which the prothrombin time condition is prolonged or the INR is elevated. Moreover, the pharmaceutical agent may be one which ameliorates, prevents or treats a condition for replenishment of one or more selected from a group consisting of blood coagulation factor II, blood coagulation factor V and blood coagulation factor VIII is effective. The pharmaceutical agent may be used to ameliorate, prevent or treat a condition for which replenishment of blood coagulation factor II and/or blood coagulation factor V is effective. This pharmaceutical agent may be used to stop bleeding and may be used in particular to prevent or stop bleeding during a surgical procedure or when the bleeding occurs due to an external injury or a disease other than a blood coagulation disorder, inclusive of cerebral hemorrhage.

The present teaching also provides a pharmaceutical agent for ameliorating, preventing or treating a condition wherein, during administration of a drug, the prothrombin time is prolonged or the international normalized ratio (INR) is elevated, the pharmaceutical agent including ribavirin or a derivative thereof. This pharmaceutical agent may include also interferon. This pharmaceutical agent may further include any selected from among selective serotonin reuptake inhibitors and selective serotonin/noradrenaline reuptake inhibitors.

The present teaching additionally provides a pharmaceutical composition for ameliorating bleeding tendencies in hemophilia patients and other patients who regularly use blood coagulants, which composition includes ribavirin or a derivative thereof.

The teaching further provides a method of screening pharmaceutical compounds for ameliorating, preventing or treating a disease or condition related to a blood coagulation factor, the method including: a step of culturing mammalian hepatocytes in the presence of a test compound; a step of detecting an amount of production of one or more selected from the group consisting of blood coagulation factor II, blood coagulation factor V and blood coagulation factor VIII produced by the hepatocytes cultured in the culturing step; and a step of selecting a test compound which exhibits a high amount of production when production of the blood coagulation factor obtained in the detection step is compared with production of the same blood coagulation factor in the absence of the test compound. In this method, in addition to these blood coagulation factors, it is also possible to detect the amount of production of blood coagulation factor VII and select a test compound while taking into account the amount of blood coagulation factor VII produced. The culturing step may be a step of culturing the hepatocytes in the presence of the test compound and interferon. Moreover, in the culture step, the test compound may be a nucleoside derivative, and may even be a ribavirin derivative.

The inventors have also discovered that, surprisingly, when ribavirin and interferon are administered for the treatment of chronic hepatitis C in patients who have hemophilia and chronic hepatitis C and who develop inhibitors in response to the administration of deficient coagulation factors, there is an effect which enables the bleeding tendency of inhibitor carriers to be ameliorated during the period of administration. The ability to ameliorate the bleeding tendencies of blood coagulation disorders in inhibitor carriers by administering ribavirin and interferon was entirely unanticipated. Moreover, the blood coagulation disorder ameliorating effects were manifested more distinctly in inhibitor carriers than in inhibitor non-carriers. Also, it was discovered that ribavirin by itself activates blood coagulation factor VII promoters, boosting the production of factor VII. Based on these findings, the present teaching may provide the following.

The teaching provides a pharmaceutical agent for ameliorating a bleeding tendency and preventing bleeding in a blood coagulation disorder patient who carries an antibody to a blood coagulation factor, which agent includes ribavirin or a derivative thereof as an active ingredient. The teaching also provides a pharmaceutical agent which is obtained by combining also interferon as an another ingredient. This combination pharmaceutical agent may be a kit composed of both a drug containing ribavirin or a derivative thereof and a drug containing interferon. Moreover, ribavirin or a derivative thereof may be administered by an oral route of administration, and interferon may be administered by one or more route of administration selected from a group consisting of intravenous, subcutaneous and intramuscular administration.

The pharmaceutical agent of the teaching may be one wherein blood collected from the patient during administration of the pharmaceutical agent has a coagulation factor VII activity of at least 75% but not more than 140%.

The pharmaceutical agent of the teaching may be adapted for use in a carrier of an antibody to blood coagulation factor VIII or IX.

### Brief Description of the Drawings

FIG. 1 is a graph plotting the change in PT% before, during and after hepatitis C treatment with ribavirin and interferon.
FIG. 2 is a graph plotting the change in INR before, during and after hepatitis C treatment with ribavirin and interferon.
FIG. 3 is a graph plotting the change in average monthly dose of coagulation factor preparations during, and both before and after, administration in Working Example 1.
FIG. 4 is a graph showing the agglutinating activity of factor VII in the serum of inhibitor carriers immediately before the start of administration ("Before treatment") and four weeks after the start of administration ("During treatment") in Working Example 2.

### Best Mode for Carrying Out the Invention

The present teaching relates to a pharmaceutical agent for ameliorating, preventing or treating diseases or symptoms associated with blood coagulation factors, and a method of screening for such agents. The inventive pharmaceutical agent for ameliorating, preventing or treating a condition in which the prothrombin time is prolonged or a condition in which the international normalized ratio (INR) is elevated, which agent includes ribavirin or a derivative thereof, or is a combination of ribavirin or a derivative thereof with interferon, is able to ameliorate, prevent or treat a condition in which the prothrombin (PT) time is prolonged or a condition in which the international normalized ratio (INR) is elevated. In other words, the pharmaceutical agent of the teaching is an agent which promotes shortening of the prothrombin time or promotes a decline in the INR.

A condition in which the PT time is prolonged or a condition in which the INR is elevated signifies here a disorder in the extrinsic and common blood coagulation systems. Surprisingly, the pharmaceutical agent of the teaching is able to increase the levels of factor II and factor V, which are common blood coagulation factors. Hence, the inventive agent is effective for ameliorating the above conditions for which replenish these blood coagulation factors that arise due to, for example, congenital or acquired deficiencies or deficits in these blood coagulation factors, is effective. Moreover, the pharmaceutical agent of the teaching can also increase the level of blood coagulation factor VII, and is thus capable of even more affectively ameliorating the above conditions.

The inventive pharmaceutical agent includes ribavirin or a derivative thereof and, through concomitant use at the time of drug administration, is able to ameliorate a condition in which the PT time is prolonged or a condition in which the international normalized ratio (INR) is elevated during such drug administration. For example, the administration of interferon or the like has the side effects of lowering the platelet level and increasing the bleeding tendency, but such a bleeding tendency can be ameliorated by the concomitant use of the inventive pharmaceutical agent.

The present teaching also relates to a pharmaceutical agent which ameliorates bleeding tendencies or prevents bleeding in blood coagulation disorder patients who carry antibodies for blood coagulation factors. By using the inventive pharmaceutical agent for ameliorating bleeding tendencies or preventing bleeding in blood coagulation disorder patients who carry antibodies for blood coagulation factors, ribavirin or a derivative thereof is administered, either alone or in combination with interferon, making it possible to ameliorate bleeding tendencies or to prevent bleeding from blood coagulation disorders in inhibitor carriers.

In addition, it is possible to reduce or extinguish the use of coagulation factor preparations in inhibitor carriers. This not only helps alleviate the financial or physical burden to the patient, it also makes it possible to avoid or check the danger of virus infections or the like arising from the administration of coagulation factor preparations, thus increasing the level of safety.

Various embodiments of the teaching arc described below in detail.

### Pharmaceutical Agent for Ameliorating. Preventing or Treating a Condition in which the PT Time is Prolonged or a Condition in which the INR is Elevated

The pharmaceutical agent of the teaching either includes ribavirin or a derivative thereof as an active ingredient, or includes ribavirin or a derivative thereof in combination with interferon as an another active ingredient.

### Ribavirin or a Derivative Thereof

The activator of the present teaching includes ribavirin or a derivative thereof. Ribavirin (1-β-D-ribofuranosyl-1,2,4-triazole-3-carboxamide) has formula (1) below. In this teaching, preferred use may be made of ribavirin.

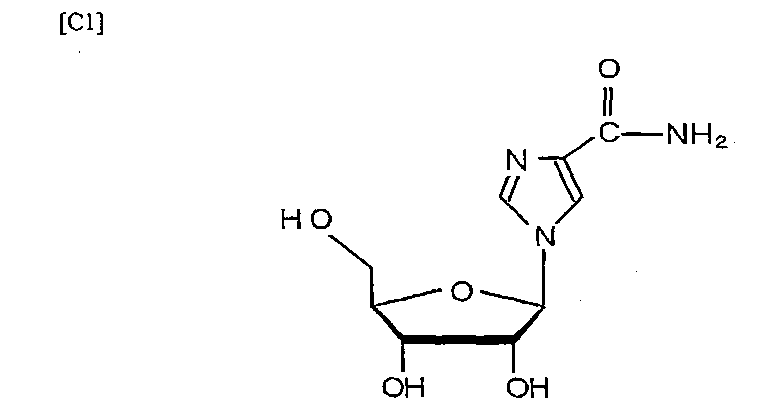

Examples of ribavirin derivatives include those in which the hydrogens of the hydroxyl groups at positions 2, 3 and 5 on ribose in the ribavirin of Formula (1) are substituted, and those in which the hydrogens on the 1,2,4-triazole group are substituted.

Examples of other ribavirin derivatives include 1-(β-D-ribofuranosyl)-1, 2 ,4-triazole disclosed in Japanese Patent Application Laid-open No. S50-154253, the nucleoside derivatives of 1,2, 4-triazole-3-carboxamide disclosed in Japanese Patent Application Laid-open No. S50-29720, and the 1, 2, 4-triazole nucleosides disclosed in Japanese Patent Application Laid-open No. S53-124271. Additional examples include the various types of ribavirin derivatives disclosed in Japanese Translation of PCT Application No. 2002-527522.

Still further ribavirin derivatives include viramidine (Antimicrobial Agents and Chemotherapy, 1872-1875 (May 2004)), the ribavirin-related compound AICAR (5-amino-1-β-D-ribofuranosylimidazole-4-carboxamide (Virus Research 107, 165-171 (2005)), and 5-ethynyl-1-β-D-ribofuranosylimidazole-4-carboxamide (EICAR). Yet other ribavirin derivatives include those shown below (J. Med. Chem. 35, 3231-3238 (1992)).

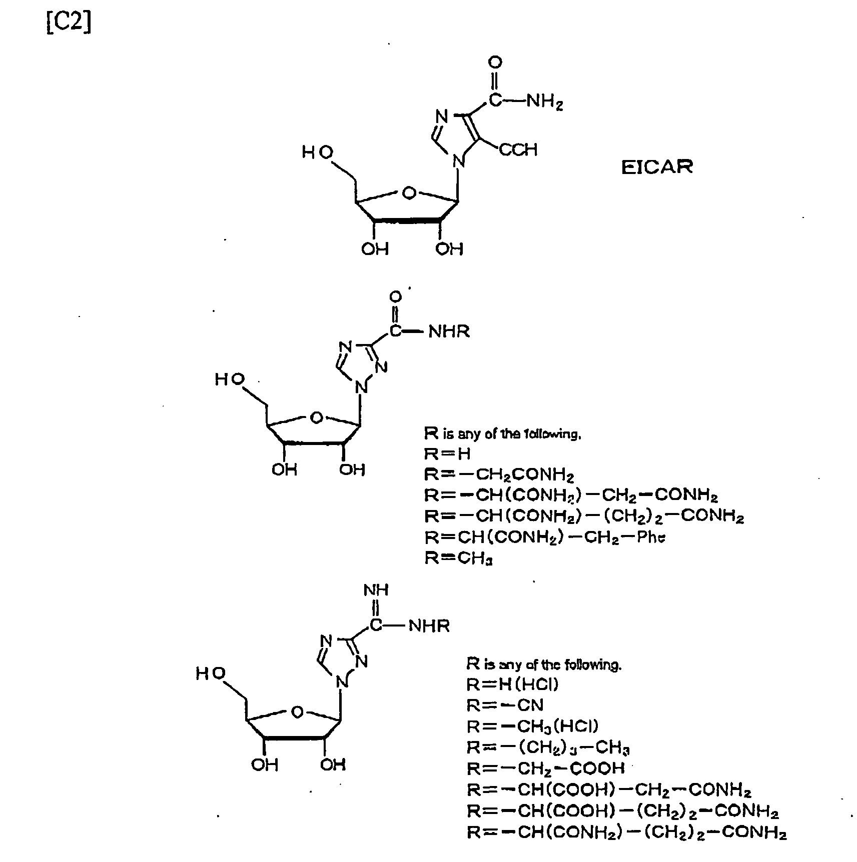

It is also possible to use a compound in which the hydroxyl group at ribose position 3 in Formula (1) has been substituted with -NH₂. Various types of substituent such as those mentioned above may be included in this compound as well. Illustrative examples of such compounds include 1-β-D-3'-amino-3'-deoxyribofuranosyl-1,2,4-triazole-3-carboxamide, 1-β-D-3'-amino-3'-deoxyribofuranosyl-1,2,4-triazole-3-carboxy hydrazide, 1-β-D-3'-amino-3'-deoxyribofuranosyl-1,2,4-triazole-3-carbohydroxamic acid, 1-β-D-3'-amino-3'-deoxyribofuranosyl-1,2,4-triarole-5-carboxamide, 1-β-D-3'-amino-3'-deoxyribofuranosyl-1,2,4-triazole-3-carboxamidrazone and 1-β-D-3'-amino-3'-deoxyribofuranosyl-1,2,4-triazole-3-carboxamidoxine (J. Med. Chem. 20, 1684-1687 (1977)).

In addition to the above, the entire contents of the patent documents and published patent applications cited in the present specification, particularly the general formulas and example compounds therein, are incorporated within the present specification.

The compatibility of each of these compounds as the pharmaceutical agent of the teaching can be easily determined by assessing the potency thereof using methods cited in this specification and in the various literature, and by assessing the toxicity, absorption, metabolism and pharmacokinetics, etc. in accordance with the knowledge of a person of ordinary skill in the art.

The ribavirin derivatives preferably have antiviral properties to various types of viruses, and more preferably have anti-viral activities against viruses for respiratory infections such as influenza, hemorrhagic fever with renal syndrome, herpes infections, Lassa fever, measles, AIDS (HIV infections), hepatitis C and hepatitis B. Anti-viral activities against viruses can be measured by suitable methods known for the target virus.

### Dosage Form of Ribavirin or Derivatives Thereof

Preparations in solid form of ribavirin or a derivative thereof include powders, tablets, dispersed granules, capsules, cachets and suppositories. Powders and tablets may contain from about 5% to about 95% of active ingredient. Suitable solid carriers are known in the field; examples include magnesium carbonate, magnesium stearate, talc, sugar and lactose. Tablets, powders, cachets and capsules may be used as suitable solid dosage forms for oral administration. Examples of pharmaceutically acceptable carriers and methods of manufacturing various compositions are cited in Remington's Pharmaceutical Sciences, 18th edition, edited by A. Gennaro, (Eaton, Pennsylvania: Mack Publishing Co., 1990).

Preparations in liquid form include solutions, suspensions and emulsions. Examples that may be cited include aqueous or aqueous-polycthylene glycol solutions for parenteral injection. Preparations in solid form may be converted to liquid preparations just prior to use for the sake of oral administration. Parenteral forms for intravenous, intramuscular or hypodermic injection are generally in the form of a sterile solution, and may include a tonicity agent (salt or glucose) and a buffer. An opacifier may be included in oral solutions, suspensions and emulsions. Preparations in liquid form encompass also solutions of nasal administration. As dosage forms of the present drug, aerosol preparations suitable for inhalation encompass both solutions and solids in powder form; in such dosage forms, combination with a pharmaceutically acceptable medium such as inert compressed air (e.g., nitrogen) is also possible. As used herein, "ribavirin or a derivative thereof" is intended to further encompass preparations in solid form, which preparations are converted just prior to use into a preparation in liquid form for oral or parenteral administration. Such liquid forms encompass solutions, suspensions and emulsions. Ribavirin or a derivative thereof may be delivered percutaneously. Percutaneous compositions may take the form of a cream, lotion, aerosol and/or emulsion, and may be included for this purpose within a matrix or reservoir-type percutaneous patch such as has hitherto been used in the field to which the teaching pertains.

Ribavirin or a derivative thereof is preferably in a single-dose form. In such a form, the preparation can be divided into unit doses of a suitable size which contain a suitable amount (e.g., an amount effective for achieving the desired purpose) of the active ingredient.

### Effective Dose of Ribavirin

The effective dose of ribavirin or a derivative thereof varies depending on the type of disease targeted, the type of compound used, the age, body weight and symptoms of the patient, and the dosage form, moreover the types of interferon and doses. For example, in the case of oral administration, ribavirin may be administered in an adult patient from one to several times daily at a daily dose within a range of preferably from about 1 mg/kg to about 200 mg/kg, more preferably from about 1 mg/kg to about 100 mg/kg, and even more preferably from about 2 mg/kg to about 40 mg/kg. Where necessary, determinations of the suitable method of administration and dose for specific circumstances may be carried out by a person of ordinary skill in the art.

### Interferon

Interferon-α may be used as the interferon. Interferon-α refers to a family of very homogenous, species-specific proteins which inhibit virus replication and cell growth, and regulate immune reactions. Preferred, non-limiting, examples of interferon-α that may be used include recombinant interferon-α-2b such as Intron-A interferon available from Schering Corporation (Kenilworth, N.J.), recombinant interferon-α-2a such as Roferon interferon available from Hoffmann-LaRoche (Nutley, N.J.), recombinant interferon-α-2c such as Berofor alpha 2 interferon available from Boehringer Ingelheim Pharmaceutical. Inc. (Ridgefield, CT), interferon-α-n1 which refers to purified mixtures of natural α-interferon such as Sumiferon available from Sumitomo (Japan), the interferon-α-n1 (INS) available as Wellferon from Glaxo-Wellcome Ltd. (London, Great Britain), the consensus α interferons mentioned in US Patent Nos. 4,897,471 and 4,695,623 (particularly Examples 7, 8 and 9 therein), and specific products available from Amgen, Inc. (Newbury Park, CA). Other preferred examples include interferon-α-n3 and interferon-α-2a or -α-2b, which are natural α interferon mixtures manufactured by Interferon Sciences and available under the trade name Alferon from Purdue Frederick Co. (Norwalk, CT). Of all interferons, interferon-α-2b is the most widely approved worldwide for the treatment of chronic hepatitis C infections. Hence, the use of interferon-α-2b is most preferred. The manufacture of interferon-α-2b is described in US Patent No. 4,530,901.

It is preferable for the interferon to be suitably modified. Although the form of modification is not limited, a polyethylene glycol-modified conjugate of interferon-α is preferred. A polyethylene glycol-modified conjugate of interferon-α-2b is more preferred, and PEG₁₂₀₀₀-interferon-α-2b is even more preferred. For example, as this compound, use may be made of a conjugate of the type manufactured in accordance with International Patent Publication No. WO95/13090, which is a conjugate having urethane linkages between the amino groups on interferon-α-2a or -2b and polyethylene glycol having an average molecular weight of 12,000.

With regard to the form of the manufactured interferon, as with the above-described manufactured form of the ribavirin or the derivative thereof, interferon may be used as a preparation in any of various solid forms or liquid forms.

### Effective Dose of Interferon

The effective dose of interferon may be suitably set according to the type of disease targeted, the type of interferon used, the age, body weight and symptoms of the patient, the dosage form, and the type and dose of ribavirin or derivative thereof with which the interferon is combined. For example, when PEG-modificd interferon-α-2b is used, administration may be carried from once to several times per week at a weekly dose within a range of from 0.1 µg/kg to about 100 µg/kg, and at a daily dose within a range of preferably from about 0.1 µg/kg to about 10 µg/kg, and more preferably from about 0.1 µg/kg to about 3.0 µg/kg. Where necessary, determinations of the suitable method of administration and dose for specific circumstances may be carried out by a person of ordinary skill in the art.

### Dosage Form

No particular limitation is imposed on the dosage form when ribavirin or a derivative thereof and interferon are administered in combination. For example, the present pharmaceutical agent may be in a form wherein both are administered at the same time as a combination drug containing ribavirin or a derivative thereof and interferon in admixture, although a dosage form other than a combination drug is preferred. That is, the present pharmaceutical agent is preferably in the form of a kit composed of both a drug containing ribavirin or a derivative thereof and a drug containing interferon.

When the inventive pharmaceutical agent is configured as a kit, the ribavirin or derivative thereof and the interferon may be administered at the same time or concomitantly, or may be administered at a fixed interval therebetween. The form in which both are administered at the same time or concomitantly is preferred, and the form in which both are administered concomitantly is more preferred.

In the case of interferon-α preparations, and especially PEG-modified interferon-α preparations, parenteral administration is preferred because oral administration is not effective. Therefore, it is preferable to combine interferon with ribavirin or a derivative thereof in a parenteral dosage form, and more preferable to combine interferon with ribavirin or a derivative thereof in an intravenous, subcutaneous or intramuscular injection form. The dosage form of ribavirin or a derivative thereof at this time may be orally administered as, for example, capsules, tablets or a liquid preparation, or may be parenterally administered via the nasal cavity as an aerosol- Oral administration is preferred.

In cases where ribavirin or a derivative thereof and interferon are used concomitantly, periods in which ribavirin or a derivative thereof is administered alone, periods in which ribavirin or a derivative thereof and interferon are administered concomitantly and periods in which interferon is administered alone, or combinations of the above periods, may be set as appropriate by the therapist.

### Uses of Pharmaceutical Agent for Ameliorating, Preventing or Treating a Condition in which the Prothrombin Time is Prolonged or a Condition in which the INR is Elevated

The pharmaceutical agent of the present teaching constituted as described above is useful, first of all, for ameliorating, preventing or treating a condition in which the prothrombin time is prolonged or a condition in which the INR is elevated. That is, the drug is able to ameliorate, prevent or treat extrinsic and shared impairment of the blood coagulating system. Examples of such impairment include congenital or acquire blood diseases that give rise to various types of anomalies in the hemostatic and/or blood coagulation systems. Typical examples include blood coagulation system disorders such as congenital or acquired hemophilia A or hemophilia B, von Willebrand disease, disseminated intravascular coagulation (DIC) and vitamin K deficiency; blood platelet disorders such as Glanzmann's thrombasthenia, thrombocytopenia, platelet abnormal function, thrombotic thrombocytopenic purpura (TTP), hemolytic uremic syndrome (HUS), idiopathic thrombocytopenic purpura (TTP), Kasabach-Marritt syndrome and Henoch-Schönlein purpura (HSP); as well as aplastic anemia, leukemia, pernicious anemia, sideroblastic anemia, Wiskott-Aldrich syndrome, chronic myeloproliferative disease, afibrinogenemia, antithrombin III deficiency, protein C deficiency, protein S deficiency, antiphospholipid antibody syndrome (APS) and dysfibrinogenemia. Further examples include hemorrhagic disease due to thrombocytopenia and decreased coagulation factors associated with HIV virus infection, or due to thrombocytopenia and decreased coagulation factors associated with hepatopathy such as liver dysfunction, hepatitis or cirrhosis of the liver resulting from other viral infections, various hepatitis viruses and other causes.

The pharmaceutical agent of the present teaching is effective for ameliorating prolonged PT time conditions or elevated INR conditions associated with virus infections or liver disease. For example, when this pharmaceutical agent is a combination of ribavirin and interferon, therapeutic effects against virus infections may appear due to the antiviral activity of the ribavirin and the virus proliferation inhibiting and other effects of interferon. Also, because a combination of ribavirin and interferon is effective against chronic hepatitis due to the hepatitis C virus, for example, therapeutic effects for hepatitis may also appear.

Illustrative examples of virus infections and diseases include influenza virus infections, parainfluenza virus infections, RS virus (RSV) infections (e.g., RSV bronchiolitis and RSV pneumonia, especially RSV infections in small children and infants, and RSV pneumonia in patients with preexisting cardiopulmonary disease), measles virus infections, Lassa fever virus infections, Korean hemorrhagic fever infections, hepatitis B virus (HBV) infections, Crimean-Congo hemorrhagic fever and HCV infections and HIV infections, encephalitis infections or Saint Louis encephalitis triggered by West Nile virus or Kunjin virus, and virus infections observed in patients having immunological disorders.

The pharmaceutical agent of the teaching may be used for ameliorating, preventing or treating a condition for which the replenishment of one or more selected from the group consisting of blood coagulation factor II, blood coagulation factor V and blood coagulation factor VIII is effective. The pharmaceutical agent of the teaching can increase the levels of all of these coagulation factors, and therefore is effective for ameliorating conditions for which replenishment of two or three of these coagulation factors at the same time is effective.

The pharmaceutical agent of the teaching increases the level of blood coagulation factor II, and therefore is effective for ameliorating, preventing or treating conditions for which blood coagulation factor II replenishment is effective. Examples of such conditions include congenital blood coagulation factor II deficiency and acquired blood coagulation factor II deficiency. Here, "blood coagulation factor deficiency" refers to a deficiency or deficit in a specific blood coagulation factor. Acquired blood coagulation factor II deficiency arise from vitamin K deficiency, liver disease and the use of anticoagulants. A deficiency of blood coagulation factor 11 also exhibits the symptoms of a prolongation in the PT time and an elevation in INR.

The pharmaceutical agent of the teaching increases the level of blood coagulation factor V, and therefore is effective for ameliorating, preventing or treating conditions for which blood coagulation factor V replenishment is effective. Examples of such conditions include congenital blood coagulation factor V deficiency. A deficiency of blood coagulation factor V also exhibits the symptoms of a prolongation in the PT time and an elevation in INR.

Because the pharmaceutical agent of the teaching is able to increase the levels of both blood coagulation factor II and factor V, it is effective for ameliorating, preventing or treating conditions for which the replenishment of both these factors is effective.

Because the pharmaceutical agent of the teaching increases blood coagulation factor VIII, it is effective for ameliorating, preventing or treating bleeding and the like in hemophilia A, wherein factor VIII is congenitally deficient, and is also able to promote activation of the blood coagulation system by blood coagulation factors II and V.

In addition, because the pharmaceutical agent of the teaching is able to increase the level of blood coagulation factor VII, which is an extrinsic coagulation factor, it is capable of effectively ameliorating, preventing or treating a prolonged PT time or elevated INR condition.

The pharmaceutical agent of the teaching can be used to stop bleeding in various situations. In particular, it can be used to prevent hemostasis during a surgical procedure, or to prevent or stop bleeding that occurs due to an external injury or due to a disease other than a blood coagulation disorder, such as a cerebral hemorrhage.

The patients in which this drug will be administered are patients which have any of the above impairments or in which there is a possibility of such impairments arising. By administration in such patients, any of the above impairments may be ameliorated, prevented or treated.

With this pharmaceutical agent, prolongation of the PT time (decrease in PT%) and elevation of INR can be suppressed or avoided in patients who have contracted a liver disease that exhibits bleeding tendencies, such as hepatitis. Given that the levels of blood coagulation factors II, V, VII and VIII have risen in the cases where ribavirin and interferon are administered concomitantly in the subsequently described working examples, the increases in these coagulation factors are thought to suppress or avert a prolongation in the PT time, and thus shorten the PT time. Moreover, given that a plurality of coagulation factors increase at the same time, such administration appears to exhibit a powerful PT time shortening effect. In addition, it is apparent from the subsequently described working examples that administering ribavirin alone improves the PT% and INR, and also that ribavirin activates coagulation factor VII promoters in hepatocytes, causing the production of factor VII to increase. This supports the fact that the administration of ribavirin or a derivative thereof alone contributes directly to improvements in PT% and INR. Given that, with respect to PT% and INR, the bleeding tendencies were improved even in cases where intcrfcron was administered in combination with ribavirin or a derivative thereof, it was apparent that interferon does not interfere with the effects of administering ribavirin or a derivative thereof alone (bleeding tendency-improving effects) and that ribavirin or a derivative thereof does not interfere with the effects of interferon. Therefore, a pharmaceutical agent obtained by combining ribavirin or a derivative thereof with interferon as the active ingredient is useful as a pharmaceutical agent capable of exhibiting the effects of each (an antiviral activity boosting effect, and a bleeding tendency improving effect along with an antiviral effect).

The pharmaceutical agent of the teaching is preferably administered in such a way that the PT% of specimens (plasma) collected from a patient during the period of administration is at least 10% but not more than 200%. At a PT% below 10%, a sufficient PT% improving effect cannot be obtained. The PT% is more preferably at least 20% but not more than 150%. It is preferable for the pharmaceutical agent of the teaching to be administered so that the INR of specimens (plasma) collected from the patient during the period of administration is at least 0.1 but not more than 5. At above 5, a sufficient INR improvement effect cannot be obtained. The INR is more preferably at least 0.7 but not more than 3.0.

### Uses of the Pharmaceutical Agent for Ameliorating. Preventing or Treating a Condition in which the PT time is Prolonged or a Condition in which the INR is Elevated During Drug Administration

The present teaching can provide a pharmaceutical agent containing ribavirin or a derivative thereof, which agent ameliorates, prevents or treats bleeding tendencies such as PT time prolongation that arise as side effects of the administration of drugs such as interferon. As mentioned above, because bleeding tendencies such as PT time prolongation can be ameliorated with a pharmaceutical agent composed of ribavirin or a derivative thereof alone or in combination with interferon, ribavirin or a derivative thereof may be utilized as a pharmaceutical agent for ameliorating bleeding tendencies such as PT time prolongation that arise as side effects during the administration of interferon and various other drugs.

When an antidepressant such as a selective serotonin reuptake inhibitor (SSRI) has been administered as the drug, bleeding tendencies are known to increase. Such bleeding tendencies due to the concomitant use of interferon and other drugs can be ameliorated with ribavirin or a derivative thereof. Therefore, pharmaceutical agents containing ribavirin or a derivative thereof can be utilized as anti-side effect medications for increased bleeding tendencies (as a side effect) during the administration of interferon and other drugs. Moreover, pharmaceutical agents containing ribavirin or a derivative thereof can be utilized as anti-side effect medications for increased bleeding tendencies (as a side effect) during the administration of, in addition to SSRIs, antidepressants such as serotoninnoradrenaline reuptake inhibitors (SNRI). In particular, the concomitant use of these antidepressants with interferon (PEG-modified interferon) is known to increase the bleeding tendencies. Hence, ribavirin or a derivative thereof can be used to prevent bleeding during the administration of interferon together with these antidepressants.

Examples of SSRIs include fluvoxamine, paroxetine, proxetine and sertraline. Examples of SNRIs include milnacipran and venlafexine.

The dose of ribavirin or a derivative thereof relative to the dose of the drug is not subject to any particular limitation. For example, the dose ofribavirin or a derivative thereof relative to the dose of interferon in the already described pharmaceutical agent which is a combination of ribavirin or a derivative thereof with interferon may be used here as the dose in which ribavirin or a derivative thereof is used with respect to the dose of interferon, or may be suitably modified. The effective dose of ribavirin or a derivative thereof can be set by a person of ordinary skill in the art based on, for example, the body weight, age, gender, degree of bleeding tendencies and serum PT time and INR of the patient.

Moreover, as with a combination drug of ribavirin or a derivative thereof and interferon, the administration of ribavirin as an anti-side effect medication during interferon administration is preferably carried out in such a way that specimens (plasma) collected from the patient during the period of interferon administration have a PT% of at least 10% but not more than 200% and an INR of at least 0.1 but not more than 5.0.

### Uses of the Pharmaceutical Agent for Ameliorating Bleeding Tendencies or Preventing Bleeding from Blood Coagulation Disorders in Inhibitor Carriers

The pharmaceutical agent of the teaching can ameliorate bleeding tendencies or prevent bleeding in blood coagulation disorder patients who carry antibodies to blood coagulation factors. The blood coagulation disorders are exemplified by the various diseases that have already been mentioned.

### Target Patients

The pharmaceutical agent of the teaching is intended for use in patients who have a blood coagulation disorder, which patients are preferably inhibitor carriers who develop antibodies to the blood coagulation factors to be replenished. The target patients are more preferably inhibitor carriers who develop antibodies to blood coagulation factor VIII or IX, and even more preferably inhibitor carriers who develop antibodies to blood coagulation factor VIII. Therapy involving the administration of deficient coagulation factors (replenishment therapy) fails to achieve sufficient ameliorative effects in inhibitor carriers, whereas the inventive pharmaceutical agent is able to reduce bleeding tendencies without the external replenishment of a blood coagulant. In addition, it is also possible to reduce or eliminate the use of coagulants other than deficient coagulation factors. Therefore, the risk of virus infection arising from the use of coagulants can be avoided, and it is also possible to reduce the burden on the patient.

The bleeding tendency ameliorating and preventing effects of the inventive pharmaceutical agent appear more clearly in inhibitor carriers than in inhibitor non-carriers. Accordingly, this pharmaceutical agent may be used in inhibitor non-carriers, although use in inhibitor carriers is especially preferred.

Inhibitor carriers in which this teaching is employed may have diseases which differ from blood coagulation disorders, such as virus infections. For example, in cases where the inventive pharmaceutical agent is a combination of ribavirin and interferon, therapeutic effects against virus infections can be manifested through the antiviral activity of ribavirin and the virus proliferation-inhibiting effect of interferon. Therefore, in cases where the inhibitor carrier has contracted a virus infection, by using this pharmaceutical agent, treatment for the virus infection can be carried out while at the same time ameliorating and preventing bleeding tendencies due to a blood coagulation disorder.

Illustrative examples of virus infections and diseases include influenza virus infections, parainfluenza virus infections, RS virus (RSV) infections (e.g., RSV bronchiolitis and RSV pneumonia, especially RSV infections in small children and infants, and RSV pneumonia in patients with preexisting cardiopulmonary disease), measles virus infections, Lassa fever virus infections, Korean hcmorrhagic fever infections, hepatitis B virus (HBV) infections, Crimean-Congo hemorrhagic fever and HCV infections and HIV infections, encephalitis infections or Saint Louis encephalitis triggered by West Nile virus or Kunjin virus, and virus infections observed in patients having immunological disorders.

This pharmaceutical agent can promote the production of coagulation factor VII in the blood of a blood coagulation disorder patient who carries inhibitors. That is, as will be apparent from the subsequently described working examples, when ribavirin or a derivative thereof and interferon are administered in an inhibitor carrier, the rise in the level of coagulation factor VII within the blood is consistent with the bleeding ameliorating and preventing effects in the inhibitor carrier. In addition, ribavirin was found to activate the blood coagulation factor VII promoter in hepatocytes and thereby increase the production of factor VII. Although these facts do not impose any limitations on the present teaching, it is conjectured that administering ribavirin or a derivative thereof to an inhibitor carrier increases coagulation factor VII in blood, and that the hemostatic effects or bleeding prevention effects in the patient increase due to the action of this increased coagulation factor VII. Moreover, because the bleeding tendencies in inhibitor carriers can be ameliorated even in cases where interferon is administered in combination therewith, it was found both that interferon docs not interfere with the effects obtained from the administration of ribavirin or a derivative thereof alone (bleeding tendency ameliorating effects) and that ribavirin or a derivative thereof does not interfere with the effects of interferon. Therefore, a pharmaceutical agent obtained by combining ribavirin or a derivative thereof and interferon as the active ingredients is useful as a pharmaceutical agent capable of exhibiting the effects of each of these active ingredients (an antiviral activity boosting effect, and a bleeding tendency ameliorating effect along with an antiviral effect).

As described above, the pharmaceutical agent of the teaching promotes the production of coagulation factor VII. Therefore, this pharmaceutical agent makes it possible to provide a novel treatment method which avoids the replenishment of coagulant in blood coagulation disorder patients requiring coagulation factor VII replenishment. In inhibitor carriers in particular, because forms of treatment that replenish coagulation factors impose additional risk of antibody development and are thus unsuitable, the present pharmaceutical agent can be advantageously used in place of replenishment therapy that uses coagulants.

To obtain the bleeding tendency ameliorating or preventing effect, it is preferable for the coagulation factor VII activity in blood collected from the patient following administration of the inventive pharmaceutical agent to be at least 40% but not more than 160%. At a coagulation factor VII activity of less than 40%, a sufficient bleeding tendency ameliorating effect or preventing effect cannot be obtained in inhibitor carriers. On the other hand, at a coagulation factor VII activity in excess of 160%, there is a risk of thrombus formation, etc. on account of the high coagulation activity. The coagulation factor VII activity in the blood is more preferably at least 75% but not more than 140%.

### Method of Screening Pharmaceutical Compounds for Ameliorating, Preventing or Treating Diseases or Conditions Related to Blood Coagulation Factors

The screening method of the present teaching includes the steps of culturing mammalian hepatocytes in the presence of a test compound; detecting an amount of production of one or more selected from among various blood coagulation factors produced by the hepatocytes cultured in the culturing step; and selecting a test compound which exhibits a high amount of production when production of the blood coagulation factor obtained in the detection step is compared with production of the same blood coagulation factor in the absence of the test compound. The screening method of the teaching enables pharmaceutical compounds containing "seed" compounds and "lead" compounds for ameliorating, preventing or treating a prolonged PT time condition or an elevated INR condition to be obtained.

The test compound used in the culturing step is not subject to any particular limitation, although this may be a nucleoside derivative. A ribavirin derivative may preferably be used as the nucleoside derivative. The test compound may be of only one type, or may be of two or more types. When two or more test compounds are used, it is preferable to use as the other test compound a type of compound which differs from nucleoside derivatives. For example, interferon may be used as the other test compound. By carrying out the culturing step in the presence of interferon, a pharmaceutical compound capable of ameliorating the prolonged PT time condition in interferon administration may be obtained.

The method of quantitatively determining the blood coagulation factor in the blood coagulation factor production amount detection step is not subject to any particular limitation. Use may be made of a known technique. The blood coagulation factors serving as the analytcs may be one or more selected from the group consisting of factor II, factor V and factor VII, and may be one or more selected from the group consisting of factor II, factor V, factor VII and factor VIII. Of these, it is preferable for the analytes to be the following extrinsic and common coagulation factors: factor II, factor V, and factor VII. Coagulation factors other than these, such as factor I, factor III, factor IV, factor IX, factor X and factor XI, may also serve as the analytes.

In the selection step, test compounds having the potential to be pharmaceutical compounds may be selected using as a guide the coagulation factors that served as the analyzes in the detection step.

The present teaching is described more fully in the following examples, which are illustrative and should not be construed as limiting the teaching.

### Working Example 1

### Preparation Administered

Ribavirin (available under the trade name Rebetol from, Schering-Plough KK) was used as the nucleoside derivative in the present teaching, and PEG interferon α-2b (available under the trade name Pegintron from Schering-Plough KK) was used as the interferon. The ribavirin was an oral preparation, and the PEG interferon α-2b was a subcutaneous injection.

These preparations were administered to the HCV positive patients shown in Table 1. In administration within this example, all the patients were given Pegintron at a dose of 1.5 µg/kg/week for 48 weeks. The ribavirin was administered orally for 48 weeks at a dose of from 600 mg/day to 1,000 mg/day in patients having a body weight of up to 60 kg. In cases where the blood hemoglobin concentration of the patient fell to below 10 g/dL due to hemolytic anemia, the ribavirin dose was decreased by 200 mg/day.

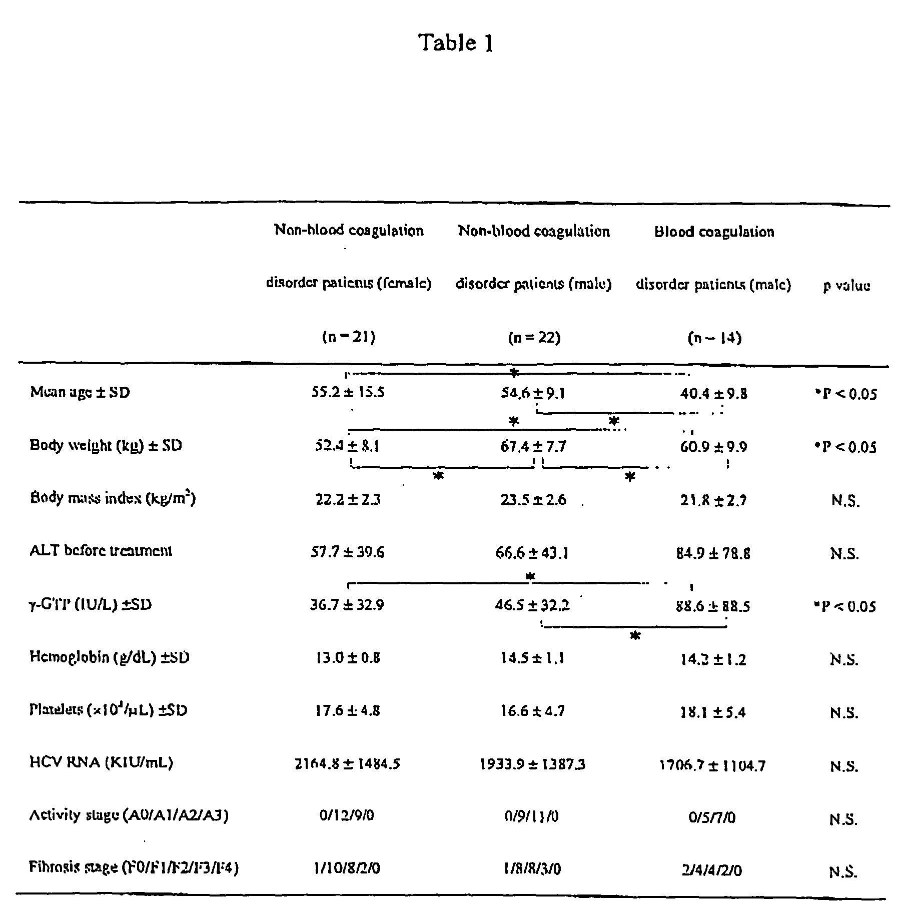

### Measurement of PT% and INR

The PT% and INR before, during and after treatment with ribavirin and interferon were measured for the patients shown in Table 1. The results are presented in Table 2 and PIGS. 1 and 2. PT% and INR measurements were each carried out by conventional methods.

**Table 2**

| | Patient group | Before Treatment | During treatment (mean values) | p value |
|---|---|---|---|---|
| | Non-blood coagulation disorder patients (female) | 105.1 ± 12.3% | 112.1 ± 16.1% | 0.0042 |
| PT | Non-blood coagulation disorder patients (male) | 98.8±183% | 106.3±16.6% | 0.0010 |
| | Blood coagulation disorder patients (male) | 96.8±15.7% | 105.7±15.0% | 0.0196 |
| | Non-blood coagulation Non-blood coaguhtion disorder patients (female) | 0.98±0.05 | 0.96 ± 0.05 | 0.0040 |
| INR | Non-blood congulation disorder patients (male) | 1.02 ±0.08 | 0.99 ±0.07 | 0.0011 |
| | Blood coagulation disorder patients (male) | 1.03±0.08 | 0.99 ±0.07 | 0.0011 |

### Measurement of Blood Coagulation Factors

Blood coagulation factors II, V, VII and VIII in plasma, both before and during treatment, were measured in some of the patients shown in Table 1 (7 of the female non-blood coagulation disorder patients, 10 of the male non-blood coagulation disorder patients, and 11 of the male blood coagulation disorder patients (9 with hemophilia and 2 with von Willebrand disease). The measurement methods used were the PT method (factor II activity, factor V activity, factor VII activity and factor X activity) and the APTT method (factor VIII activity). The results are presented in Table 3.

**Table 3**

| | Before treatment | During treatment* |
|---|---|---|
| Factor II | 82.61 | 87.32 |
| Factor V | 90.64 | 104.7 |
| Factor VII | 91.36 | 100.2 |
| Factor VIII | 82.26 | 97.46 |
| Factor X | 95.07 | 92.93 |

| | | |
|---|---|---|
| *Four weeks after the start of treatment | | |

In addition, the results of PT% and INR measurements before, during and after treatment for the group of patients shown in Table 3 are presented in Table 4.

**Table 4**

| Test | Before treatment | During treatment* | After treatment |
|---|---|---|---|
| PT% | 100.6 | 108.1 | 99.351 |
| INR | 1.009 | 0.976 | 1.01 |

| | | | |
|---|---|---|---|
| *Four weeks after the start of treatment | | | |

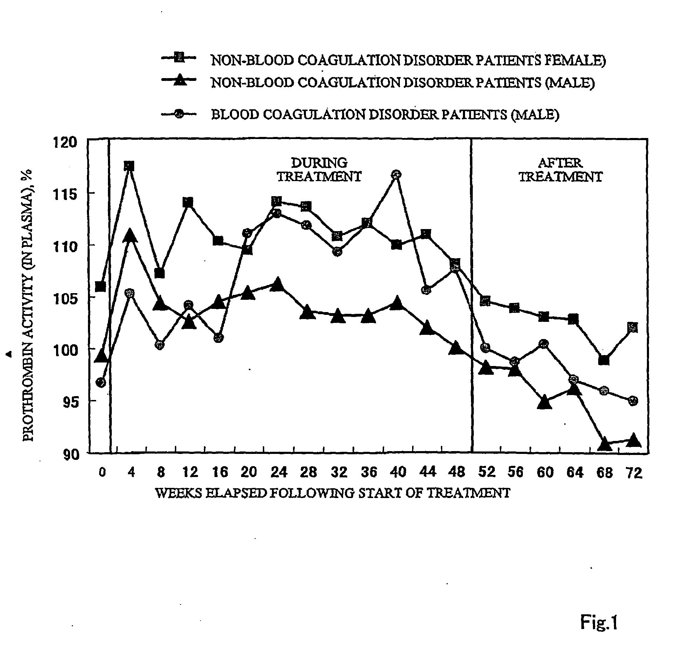

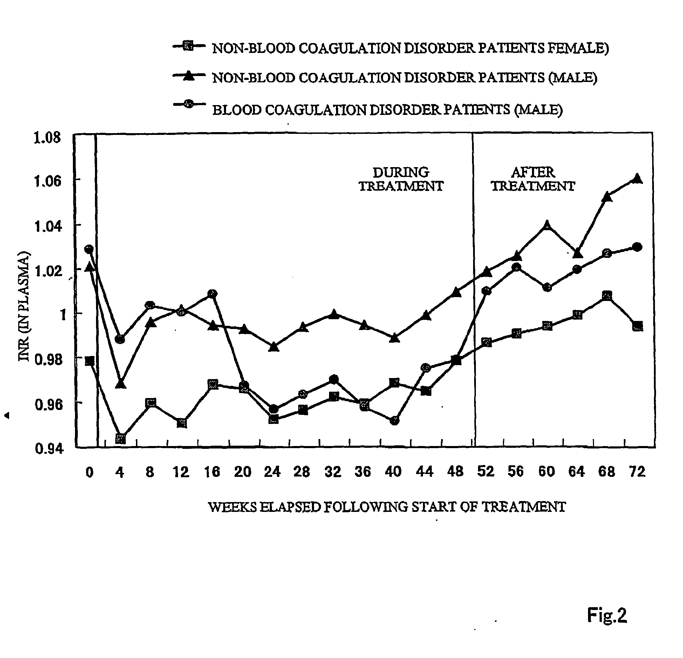

As shown in FIGS. 1 and 2, the PT% rose and the INR fell for all the patient groups (female non-blood coagulation disorder patients, male non-blood coagulation disorder patients, male blood coagulation disorder patients) during treatment as compared with before and after treatment. Generally, during interferon administration, the platelets decrease and the bleeding tendency increase. However, when interferon was used together with ribavirin, tendencies opposite to a bleeding tendency were found to appear. Moreover, as shown in Table 2, the PT% during treatment was significantly elevated compared with before treatment, and the INR during treatment was significantly lower compared with before treatment.

Also, as shown in Table 3, the coagulation factor activity during treatment was elevated for coagulation factors other than factor X.

In addition, as shown in Table 4, for the patient groups in Table 3, tendencies for the PT% to be elevated and the INR to be depressed during treatment and for the PT% to be depressed and the INR to be elevated following treatment were observed.

As is apparent from the above results, the PT% rose and the INR fell during the period of treatment with both ribavirin and interferon. That is, administering a compound such as ribavirin and interferon in combination was found to have effects such as elevating the PT% not only in patients with both a blood coagulation disorder and liver disease, but also in liver disease patients without a blood coagulation disorder, thus demonstrating that this type of bleeding tendency can be ameliorated.

At the same time, because interferon administration is known to generally lower the platelet count and give rise to bleeding tendencies, the concomitant use of a compound such a ribavirin when administering interferon was found to be effective as an anti-side effect medication for ameliorating aggravated bleeding tendencies as a side effect.

### Working Example 2

This example is a case in which ribavirin alone was administered in a hemophilia A patient. Patient A (29-year-old male, HTV positive, with chronic hepatitis C (type 2b, 5000 KIU/mL before treatment), and having undergone virus eradication (sustained virologic response, or "SVR") with interferon + ribavirin treatment), after a fixed period of time following confirmation ofSVR, was given 600 mg/day of ribavirin alone. The dose of coagulant averaged 3,167 units/month before treatment, and decreased to an average of 2,000 units/month after treatment.

### Working Example 3

In this example, the dose of coagulant used in a case where ribavirin alone was administered to a hemophilia A patient was evaluated. Patient B (36-year-old male with chronic hepatitis C (type 1 a, 280 KIU/mL before treatment), and having undergone virus eradication (SVR) with interferon + ribavirin treatment), after a fixed period of time following confirmation of SVR, was given 600 mg/day of ribavirin alone. The dose of coagulant averaged 9,333 units/month before treatment with ribavirin alone, and decreased to an average of 3,333 units/month after treatment.

### Working Example 4

In this example, the PT% and INR were evaluated in a case where 2T (10 mg)/day of an SSRI (trade name, Paxil) was administered as an antidepressant during the concomitant administration of interferon and ribavirin. The patient (57-year-old female; type 2a chronic hepatitis C, 188 KIU/mL before start of treatment; depression; Sjögren syndrome) was given 400 mg/day of ribavirin and 60 µg/week ofPegintron for 24 weeks. The PT% and INR before the start of treatment were respectively 114.7% and 0.94. When 4 weeks had elapsed following the start of treatment, the PT% and INR were respectively 131.1% and 0.89. Hence, clear improvements occurred in both values. During the period of administration, the patient experienced no fever, exacerbation of the depression or bleeding symptoms.

### Working Example 5

Based on a DNA sequence in the human coagulation factor VII promoter region (Accession No.: AL137002), healthy human genomic DNA as the template was amplified by the PCR process (sense primer: 5'-ACTTGAACCCGGGAGGTG-3'; antisense primer: 5'-GGAaAgCtTGATGAAATCTCTGCAGT-3'; changes were made at the lower case letters, introducing a Hind III site (underlined portion)), thereby obtaining a DNA fragment containing this promoter region. This 722 bp promoter DNA fragment was subcloned by a TA cloning process in the (+) direction at the EcoRV site of pBluescript II KS(+), following which a plasmid having DNA which codes for luciferase protein under the operation of this promoter was constructed with the Hind III fragment using a luciferase cDNA sequence-carrying plasmid (pGVB2U from pGL3-Basic available from Promega; supplied by Professor Kokame of the National Cardiovascular Center (Kokame et al., JBC 276, 9166-9205 (2001))). This plasmid and the β-galactosidase expression vector pSV-β-galactosidase plasmid (available from Promega) for correcting the gene transfer efficiency were transfected by the calcium sedimentation method into cultured human hepatocytes (HepG2 cells), thereby obtaining transformed cultured human hepatocytes in a transient expression system.

These transformed cultured human hepatocytes (70 to 80% confluent) were cultured at 5% CO₂ and 37°C for 40 hours in 10% FCS + DMEM, both in the presence and absence ofribavirin, following which the cells were recovered, then lysed with the PikaGene cultured cell lysis agent LCβ-PGC-51 (Toyo Ink Mfg Co., Ltd.). The luciferase activity in each cell lysate was measured by reaction with a PikaGene luminescent substrate luciferase assay (Toyo Ink Mfg Co., Ltd.), and the amount of luminescence was measured with a Mini Lumat LB9506 luminometer (Berthold). Similarly, the β-gal activity was determined by reaction with 2x Assay Buffer (Promega), and measurement of the light absorbance. Analysis of the relative rise in luciferase activity, as corrected by the measured β-gal activity, showed a 1.6-fold rise in luciferase activity in a medium containing 50 µg/mL of ribavirin compared with ribavirin-free medium; in a medium containing 150 µg/mL of ribavirin, the relative rise in luciferase activity was 3.6-fold.

From the above results, ribavirin was found to act on blood coagulation factor VII promoters in hepatocytes, causing the production of factor VII to be increased.

### Working Example 6

### Preparation Administered

Ribavirin (available under the trade name Rebetol from, Schering-Plough KK) was used as the nucleoside derivative in the present teaching, and PEG interferon α-2b (available under the trade name Pegintron from Schering-Plough KK) was used as the interferon. The ribavirin was an oral preparation, and the PEG interferon α-2b was a subcutaneous injection.

These preparations were given to the hemophilia patients who were also HCV positive shown in Table 1. Of the patients shown in Table 4, Patient No. 6 had been given interferon-α-2b alone prior to administration in this example, but the HCV was not eradicated. In administration within this example, all the patients were given 1.5 µg/kg of Pegintron once per week for 48 weeks. At the same time, ribavirin was orally administered at a dose of 600 mg/day in patients with a body weight up to 60 kg, at a dose of 800 mg/day in patients with a body weight greater than 60 kg and up to 80 kg, and at a dose of 1,000 mg/day in patients with a body weight greater than 80 kg. When the hemoglobin concentration in the patient's blood fell below 10 g/dL due to hemolytic anemia, the ribavirin dose was lowered to just 200 mg/day. In cases where the hemoglobin concentration fell below 8.5 g/dL, ribavirin administration was stopped.

**Table 5**

| Patient No. | Age | Type of hemophilia | Severity | HCV-RNA level (KIU/mL) | Genotype | Ribavirin dose (mg/d) | HCV eradication | Inhibitor presence | HIV infection state |
|---|---|---|---|---|---|---|---|---|---|
| 1 | 24 | A | severe | 3,400 | 1 | 800 | no | yes | N |
| 2 | 35 | B | severe | 1,100 | 4a | 800 | yes | no | P |
| 3 | 43 | A | severe | 991 | 1b | 800 | yes | no | N |
| 4 | 41 | A | severe | 2,860 | 1a | 600 | yes | no | N |
| 5 | 34 | A | severe | 15.4 | 1b | 600 | yes | no | N |
| 6 | 36 | A | severe | 941 | 1a | 600 | - | no | N |
| 7 | 22 | A | severe | 2,360 | 1b | 800 | - | no | N |

In Table 5, the severity of hemophilia was rated as mild when the level of coagulation factors exceeded 5%, moderate when this level was from 1 to 5%, and severe when this level was below 1%. The HCV-RNA level was measured with an Amplicor HCV Assay, version 2 (Roche) at the start of administration. The genotype of the HCV virus was determined from the base sequence of the 5'-UTR region thereof. HCV eradication was indicated as "yes" when HCV-RNA viruses were not detected for 24 weeks following the end of administration. The HIV infection state was determined by the HIV antibodies using a particle agglutination assay kit (Fujirebio Inc.).

### Amount and Frequency of Coagulation factor Preparation Use

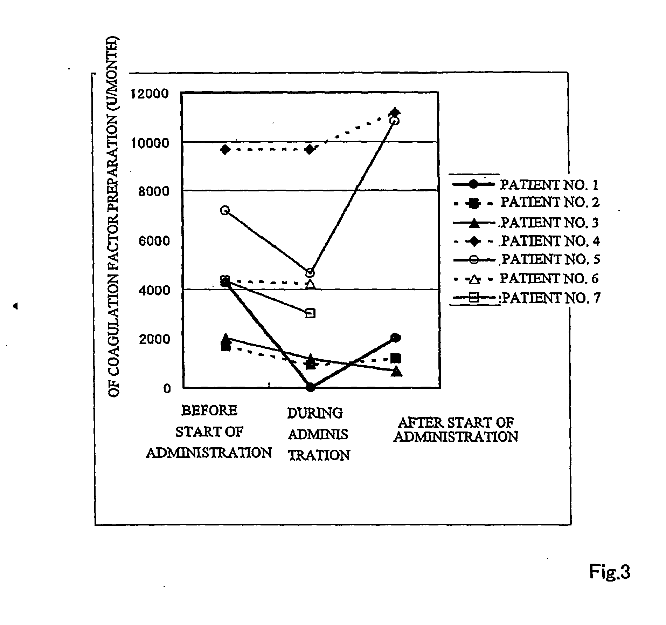

The amount and frequency of use of coagulation factor preparation was evaluated from the records of the patients themselves. The amount of coagulation factor preparation used (average values) before, during and after the end of the administration described above in seven patients with severe hemophilia in Table 1 was compared. The coagulation factor preparations used were as follows: a coagulation factor VII preparation was used in Patient No. 1, a coagulation factor IX preparation was used in Patient No. 2, and coagulation factor VIII preparations were used in Patient Nos. 3 to 7. Significant differences at P < 0.05 were determined by a t-test (paired). In addition, interviews were carried out on the patient's daily lives. The results are presented in Table 6 and FIG. 3.

**Table 6**

| Patient No. | Age | Type of hemophilia | Inhibitor presence | Amount of coagulation factor preparation used (U/month) | | |
|---|---|---|---|---|---|---|
| | | | | Before administration | During administration | After administration |
| 1 | 24 | A | yes | 4,250.0 | 0.0 | 2,000.0 |
| 2 | 35 | B | no | 1,666.7 | 909.1 | 1,166.7 |
| 3 | 43 | A | no | 2,000.0 | 1,182.0 | 666.7 |
| 4 | 41 | A | no | 9,666.7 | 9,666.7 | 11,166.7 |
| 5 | 34 | A | no | 7,166.7 | 4,636.5 | 10,833.3 |
| 6 | 36 | A | no | 4,333.3 | 4,200.0 | -- |
| 7 | 22 | A | no | 4,333.3 | 3,000.0 | -- |

In Table 6, the amount of coagulation factor preparation used during the period of administration is shown as the average value for 48 weeks of administration in Patient Nos. 1 to 6, and is shown as the average value for four months of administration in Patient No. 7. Also, the amount of coagulation factor preparation (average value) following the period of administration is shown as the average value for six months following administration in Patent Nos. 2 to 5, and is shown as the average value for five months following administration in Patent No. 1.

As shown in Table 6 and FIG. 3, in the six patients who required coagulation factor preparations and did not carry inhibitors (Nos. 2 to 7), the average amount of coagulation factor preparation used in the six months prior to the period of combined administration was 4,861.1 U (standard deviation, 3,077.6), the average amount of use during the period of administration was 3,932.4 U (standard deviation, 3,194.5), and the average amount of use in the six months following the end of administration was 5,958.4 U (standard deviation, 5,826.7). The average monthly amount of use during the period of administration was significantly lower than the amount of use prior to the period of administration (P < 0.05).

In Patient No. 1, who requires coagulation factor preparation and also carries inhibitors, the average monthly amount of use in the six months prior to the period of combined administration was 4,250 U. During the 48 weeks of administration, coagulation factor preparation was not used a single time. In the five months following the end of administration, the average monthly amount of use was 2,000 U.

As is apparent from the above results, during the period of ribavirin and interferon administration, the amount and frequency of coagulation factor preparation use dramatically decreased, indicating a decline in bleeding tendencies. In the inhibitor carrier in particular, the bleeding tendency during this period of administration decreased even more dramatically than in inhibitor non-carriers, so that the use of coagulation factor preparation.was not required. This suggests that the concomitant administration of ribavirin and interferon is especially effective for ameliorating bleeding tendencies in inhibitor carriers.

The above shows that, by administering a combination of a compound such as ribavirin with interferon, bleeding tendencies can be lowered in hemophilia patients who carry inhibitors without the external replenishment of coagulation factor preparation. As a result, it was found that the risk of additional antibody development arising from replenishment therapy in inhibitor carriers can be avoided and various burdens on the patient can be alleviated, in addition to which the danger of virus infection from replenishment therapy can be avoided or suppressed.

### Measurement of Factor VII

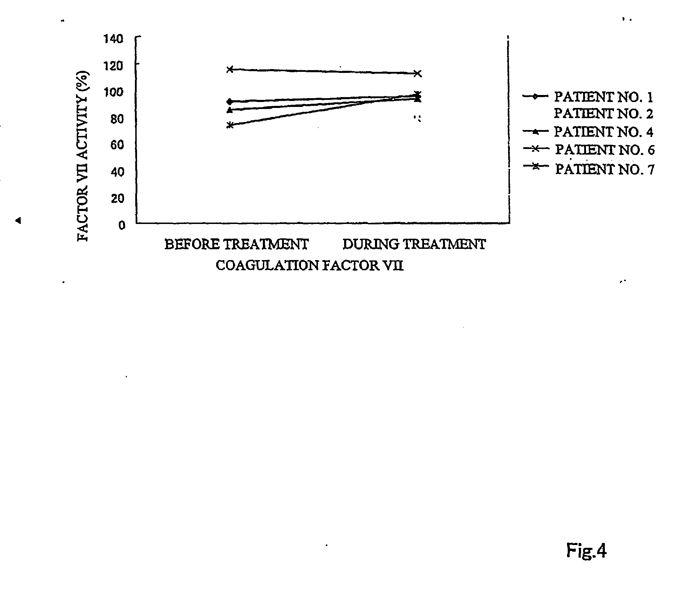

The factor VII activity in the patient's serum (specimens: citrated plasma; test method: PT method) was measured. Measurement was carried out using plasma collected within one week prior to the start of ribavirin and interferon administration, and using plasma collected four weeks after the start of administration. The results are presented in Table 7and FIG. 4.

**Table 7**

| Patient No. | Factor VII (before administration) | Factor VII (during administration) |
|---|---|---|
| 1 | 91 | 95 |
| 2 | 77 | 78 |
| 3 | - | - |
| 4 | 85 | 93 |
| 5 | - | - |
| 6 | 115 | 112 |
| 7 | 73 | 96 |

### (units: %)

As shown in Table 7 and FIG. 4, of the patients in which factor VII was measured (Patient Nos. 1, 2, 4, 6 and 7), the factor VII level increased in all but one patient. In Patient No. 1, who was an inhibitor carrier, the increase was about 4%. Because it has been pointed out that an excessive rise in factor VII increases the risk to the cardiovascular system, even an increase of about several percent was presumed to contribute significantly to the amelioration of bleeding tendencies. Aside from one patient (Patient No. 5), factor VII showed an increasing tendency. However, in the inhibitor carrier, this bleeding tendency ameliorating effect (reduction in amount of coagulant use) was pronounced.

As shown above, the rise in factor VII around the start of administration is consistent with the decline in bleeding tendencies (warfarin resistance) during ribavirin administration in Working Example 1. Hence, an increase in factor VII appears to lower bleeding tendencies in inhibitor carriers.

### TEXT IN SEQUENCE LISTING

SEQ ID NOS: 1 and 2: primers

## Claims

1. A pharmaceutical agent comprising ribavirin or a derivative thereof as an active ingredient, the pharmaceutical agent ameliorating, preventing or treating a condition in which a prothrombin time is prolonged or a condition in which an international normalized ratio (TNR) is elevated.

2. The pharmaceutical agent according to claim 1, which is obtained by further combining interferon as an active ingredient.

3. The pharmaceutical agent according to claim 1 or 2, which ameliorates, prevents or treats a condition in association with liver disease in which the prothrombin time is prolonged or the INR is elevated.

4. The pharmaceutical agent according to any of claims 1 to 3, which ameliorates, prevents or treats a condition for which replenishment of one or more selected from a group consisting of blood coagulation factor II, blood coagulation factor V and blood coagulation factor VIII is effective.

5. The pharmaceutical agent according to claim 4, which ameliorates, prevents or treats a condition for which replenishment of one or more selected from a group consisting of blood coagulation factor II and blood coagulation factor V is effective.

6. The pharmaceutical agent according to any of claims 1 to 5, which is to stop bleeding.

7. The pharmaceutical agent according to claim 6, which is to prevent or stop bleeding during a surgical procedure or when the bleeding occurs due to an external injury or a disease other than a blood coagulation disorder, inclusive of cerebral hemorrhage.

8. A pharmaceutical agent for ameliorating, preventing or treating a condition in which, during administration of a drug, a prothrombin time is prolonged or an international normalized ratio (INR) is elevated,
the pharmaceutical agent comprising ribavirin or a derivative thereof.

9. The pharmaceutical agent according to claim 8, which comprises interferon.

10. The pharmaceutical agent according to claim 8 or 9, wherein the drug includes any selected from among selective serotonin reuptake inhibitors and selective serotonin/noradrenaline reuptake inhibitors.

11. A method of screening pharmaceutical compounds for ameliorating, preventing or treating a disease or symptom related to a blood coagulation factor,
the method comprising:
a step of culturing mammalian hepatocytes in the presence of a test compound;
a step of detecting an amount of production of one or more selected from a group consisting of blood coagulation factor II, blood coagulation factor V and blood coagulation factor VIII produced by the hepatocytes cultured in the culturing step; and
a step of selecting a test compound which exhibits a high amount of production when production of the blood coagulation factor obtained in the detection step is compared with production of the same blood coagulation factor in the absence of the test compound.

12. The method according to claim 11, wherein the culturing step is a step of culturing the hepatocytes in the presence of the test compound and interferon.

13. The method according to claim 11 or 12, wherein the test compound is a nucleoside derivative.

14. The method according to claim 13, wherein the nucleoside derivative is a ribavirin derivative.

15. A pharmaceutical agent for use in ameliorating a bleeding tendency and preventing bleeding in a blood coagulation disorder patient who carries an antibody to a blood coagulation factor,
the agent comprising ribavirin or a derivative thereof as an active ingredient.

16. The pharmaceutical agent according to claim 15, which is obtained by further combining interferon as an active ingredient.

17. The pharmaceutical agent according to claim 16, wherein the agent is a kit comprising both a drug containing ribavirin or a derivative thereof and a drug containing interferon.

18. The pharmaceutical agent according to any of claims 15 to 17, wherein blood collected from the patient during administration of the pharmaceutical agent has a coagulation factor VII activity of at least 75% but not more than 140%.

19. The pharmaceutical agent according to any of claims 15 to 18, which is for use in a carrier of an antibody to blood coagulation factor VIII or IX.


SEQUENCE LISTING

<110> National Corporation University of Nagoya University

<120> Use of Rivabirin for Blood Clotting Disorders

<130> K08-297-PCT

<160> 2

<170> PatentIn version 3.3

<210> 1
<211> 18
<212> DNA
<213> Artificial

<220>
<223> primer

<400> 1
acttgaaccc gggaggtg 18

<210> 2
<211> 25
<212> DNA
<213> Artificial

<220>
<223> primer

<400> 2
ggaaagcttg atgaaatctc tgcag 25